Europäisches Patentamt

European Patent Office

Office européen des brevets

(19)

(11) **EP 1 360 469 B1**

(12) **FASCICULE DE BREVET EUROPEEN**

(45) Date de publication et mention
de la délivrance du brevet:
**24.11.2004 Bulletin 2004/48**

(51) Int Cl.⁷: **G01N 1/40**, H01L 21/00,
C01B 33/107

(21) Numéro de dépôt: **02704803.2**

(22) Date de dépôt: **12.02.2002**

(86) Numéro de dépôt international:
**PCT/FR2002/000521**

(87) Numéro de publication internationale:
**WO 2002/065097 (22.08.2002 Gazette 2002/34)**

(54) **PROCEDE DE DOSAGE D'ELEMENTS DANS UN SUBSTRAT POUR L'OPTIQUE, L'ELECTRONIQUE OU L'OPTOELECTRONIQUE**

VERFAHREN ZUR DOSIERUNG VON ELEMENTEN AUS EINEM SUBSTRAT FÜR OPTIK, ELEKTRONIK ODER OPTOELEKTRONIK

METHOD FOR MEASURING ELEMENTS IN A SUBSTRATE FOR OPTICS, ELECTRONICS OR OPTOELECTRONICS

(84) Etats contractants désignés:
**AT BE CH CY DE DK ES FI FR GB GR IE IT LI LU MC NL PT SE TR**

(30) Priorité: **13.02.2001 FR 0101926**

(43) Date de publication de la demande:
**12.11.2003 Bulletin 2003/46**

(73) Titulaire: **S.O.I.Tec Silicon on Insulator Technologies**
**38190 Bernin (FR)**

(72) Inventeur: **VIRAVAUX, Laurent**
**F-38120 Le Fontanil (FR)**

(74) Mandataire: **Martin, Jean-Jacques et al**
**Cabinet Régimbeau**
**20, rue de Chazelles**
**75847 Paris Cedex 17 (FR)**

(56) Documents cités:
**EP-A- 0 450 558          US-A- 3 273 963**
**US-A- 4 990 459          US-A- 6 053 984**
**US-A- 6 164 133**

EP 1 360 469 B1

**Description**

L'invention concerne un procédé de dosage d'un élément dans un matériau comprenant du silicium, ce matériau étant destiné notamment à la fabrication de composants pour l'optique, l'électronique ou l'optoélectronique. Par exemple, l'invention concerne le dosage d'éléments métalliques susceptibles de contaminer des substrats de silicium.

Dans la technologie du silicium, la pièce maîtresse, en micro-électronique, est la capacité MOS (Métal / Oxyde / Semi-conducteur). La structure d'une capacité MOS est constituée de deux électrodes (par exemple l'une en métal ou en silicium polycristallin et l'autre en silicium monocristallin), séparées par une fine couche isolante d'oxyde de silicium ($SiO_2$) appelé diélectrique ou oxyde de grille. L'oxyde de grille est réalisé par oxydation thermique, la croissance se fait en surface et en profondeur. Les oxydes thermiques sont des matériaux vitreux (ou amorphes) ne présentant pas un ordre cristallographique à longue distance, mais simplement une homogénéité chimique et un ordre à courte distance. Seulement 43 % de l'espace du réseau cristallin est occupé, favorisant ainsi les mécanismes de piégeage et de diffusion. La présence d'impuretés introduit des espèces étrangères qui viennent se placer en sites interstitiels ou substitutionnels. Ces impuretés sont principalement des ions alcalins (calcium, potassium, sodium) et des ions métalliques (fer, aluminium, zinc, cuivre, nickel ...).

Si un substrat présentant de telles impuretés, au niveau de la couche de silicium actif, est porté à haute température, ces impuretés peuvent précipiter et conduire ainsi à la formation de défauts cristallins.

La diffusion des impuretés va provoquer une ségrégation des espèces et de fortes concentrations localisées. Celles-ci agissent comme des pièges pour les porteurs de charge, en fournissant des niveaux d'énergie qui facilitent les recombinaisons électron-trou. Ces impuretés dégradent les performances électriques, ou entraînent le claquage des futurs circuits. L'épaisseur des oxydes de grille peut actuellement atteindre des épaisseurs de quelques dizaines d'angströms seulement . Une couche de 50 Å par exemple correspond à une vingtaine de couches d'atomes. Cette faible épaisseur exacerbe l'impact des défauts. Donc la qualité de cette couche est intimement liée au procédé d'oxydation mais aussi à la qualité du substrat sur lequel il va croître. Pour certaines applications de la micro-électronique, on forme une couche d'oxyde sous une couche de semi-conducteur. On appelle une telle couche d'oxyde, un oxyde enterré.

On utilise des oxydes enterrés par exemple lorsqu'on réalise des substrats SOI (SOI est l'acronyme de l'expression anglo-saxonne « Silicon On Insulator », c'est à dire « Silicium Sur Isolant » en français).

Ce type de substrats peut être utilisé pour réaliser un circuit électronique sur du silicium isolé par un isolant, ici l'oxyde enterré, d'un support mécanique.

Dans un oxyde enterré, la présence de contaminants dégrade l'isolation électrique entre le circuit et le support mécanique. Il est donc important de doser ces impuretés.

Par ailleurs, de nombreux instruments constitués d'un matériau comprenant une forte concentration de silicium, tel que le verre, le quartz, etc., sont utilisés dans des procédés de fabrication de substrats et de composants pour la microélectronique, l'optique ou l'optoélectronique. Les performances de ces substrats et de ces composants dépendent en particulier des impuretés qu'ils contiennent. Or, les instruments avec lesquels ils sont manipulés (telles que des nacelles de quartz pour substrats par exemple) peuvent les contaminer. Donc ici encore, on peut souhaiter doser les contaminants de ces instruments.

On connaît déjà de nombreuses techniques pour doser des éléments, telles que des impuretés, dans un matériau.

Certaines de ces techniques ne nécessitent pas de préparation de l'échantillon. Ce sont, par exemple, la spectrométrie de masse des ions secondaires (connue de l'homme du métier sous l'acronyme SIMS de l'expression anglo-saxonne « Secondary Ion Mass Spectrometry ») et la fluorescence X totale (connue de l'homme du métier sous l'acronyme TXRF de l'expression anglo-saxonne « Total X Ray Fluorescence »).

D'autres techniques nécessitent une préparation de l'échantillon correspondant par exemple à une simple mise en solution. C'est le cas notamment de la spectrométrie d'absorption atomique (connue de l'homme du métier sous l'acronyme AAS de l'expression anglo-saxonne « Atomic Absorption Spectrometry »), et parfois de la TXRF également.

Lorsqu'on prépare des échantillons destinés à un dosage, on doit éviter d'ajouter (contamination opératoire) ou de soustraire (précipitation, adsorption, etc.) des contaminants.

Des techniques connues de préparation d'échantillons pour de tels dosages consistent à graver chimiquement la surface d'un substrat.

Un de ces techniques est connue de l'homme du métier sous l'acronyme VPD de l'expression anglo-saxonne « Vapor Phase Decomposition». Une autre de ces techniques est connue de l'homme du métier sous l'acronyme LPD de l'expression anglo-saxonne « Liquid Phase Decomposition ».

La technique VPD consiste à graver de l'oxyde de silicium, avec des vapeurs d'acide fluorhydrique. Cette réaction se passe généralement à température ambiante et peut être facilitée par un refroidissement du matériau à graver, pour favoriser la condensation des vapeurs d'acide fluorhydrique sur ce matériau.

La gravure s'effectue selon la réaction suivante :

$$SiO_2 \text{ (s)} + 6 \text{ HF (aq)} \rightarrow H_2SiF_6 \text{ (aq)} + 2 \text{ H}_2O \tag{I}$$

Cette technique peut être en outre utilisée pour décomposer de l'oxyde natif, typiquement plusieurs dizaines d'angstrôms, sur du silicium ou des couches d'oxyde plus épaisses (par exemple plusieurs centaines d'angströms) formées volontairement par exemple au cours d'une oxydation thermique.

La technique LPD consiste à graver en phase liquide.

Cette technique peut être utilisée pour graver aussi bien du silicium que de l'oxyde de silicium.

Pour graver par cette technique des oxydes épais, typiquement quelques milliers d'angströms à quelques microns, voire quelques dizaines de microns, obtenus par exemple par dépôt d'oxyde, on peut utiliser une solution d'acide fluorhydique (HF).

Pour graver, toujours par cette technique, du silicium non oxydé par exemple, on utilise un mélange d'acide nitrique (HNO$_3$) et d'acide fluorhydrique (HF). La réaction sur le silicium est alors la suivante :

$$Si + HNO_3 + 6 \text{ HF} \rightarrow H_2SiF_6 + NO_2 + H_2O + H_2$$

Au cours de cette dernière gravure, plusieurs réactions simultanées interviennent : le silicium est oxydé par l'acide nitrique et l'oxyde de silicium est gravé par l'acide fluorhydrique.

Selon ces deux techniques de gravure, en phase vapeur et en phase liquide, les différents éléments initialement présents dans l'oxyde de silicium ou le silicium sont également passés en solution.

D'autres techniques de dosage que l'AAS et/ou la TXRF ne peuvent se satisfaire de ces seules techniques de décomposition. Tel est le cas par exemple d'une analyse par spectrométrie de masse à plasma inductif (connue de l'homme du métier sous l'acronyme ICPMS, de l'expression anglo-saxonne « Inductively Compled Plasma Mass Spectrometry) qui a notamment pour mérite une rapidité accrue comparativement aux techniques pré-citées. En effet, la solution obtenue à l'issue de ces préparations tant en phase liquide, qu'en phase vapeur, lorsque l'épaisseur à graver est importante, a une concentration en silicium typiquement égale à quelques grammes par litre, ce qui représente une concentration trop élevée pour les équipements ICPMS actuels (saturation de l'appareil d'analyse, notamment pour ce qui est de l'ionisation des éléments). La viscosité de la solution obtenue est également incompatible avec les équipements ICPMS actuels. Dans ce cas, des techniques de préparation de l'échantillon plus poussées sont requises.

US6164133 décrit un procédé de dosage d'au moins un élément dans un matériau comprenant du silicium comprenant la décomposition d'au moins une partie du matériau avec un agent d'attaque tel qu'un mélange d'acide nitrique et fluorhydrique (HF) pour former une solution contenant les éléments à doser, le chauffage de cette solution pour obtenir une solution conservant la quantité de chaque élément à doser, la vaporisation de la solution et la remise en solution du résidu sec avant le dosage par ICPMS. L'étape de décomposition implique la formation d'acide hexafluorosilicique et le chauffage puis la vaporisation de la solution obtenue impliquent la formation de tetrafluorure de silicium gazeux.

Un but de l'invention est d'obtenir une technique de dosage permettant d'éviter les inconvénients des méthodes précitées tout en conservant une bonne précision dans le dosage des éléments en faible concentration, voire en améliorant la précision des dosages.

Ce but est atteint, selon l'invention, grâce à un procédé de dosage d'au moins un élément dans un matériau comprenant du silicium et utilisé dans la fabrication de substrats ou de composants pour l'optique, l'électronique ou l'optoélectronique, caractérisé en ce qu'il comprend les étapes suivantes:

(a) décomposer au moins une partie du matériau avec un agent d'attaque pour former une solution contenant de l'acide hexafluorosilicique et les éléments à doser,

(b) chauffer cette solution jusqu'à une température inférieure à la température d'ébullition de l'acide hexafluorosilicique telle qu'au moins une partie substantielle de l'acide hexafluorosilicique soit transformée en tétrafluorure de silicium et qu'une partie substantielle du tétrafluorure de silicium s'évapore, pour obtenir une solution pour dosage dans laquelle la teneur en silicium soit sensiblement réduite tout en conservant la quantité de chaque élément à doser, et

(c) doser au moins un élément contenu dans la solution pour dosage.

L'étape (b) du procédé met en jeu la réaction suivante :

$$H_2SiF_6 \; (I) \rightarrow SiF_4 \; (g) + 2HF$$

Grâce à cette réaction, on élimine une grande partie du silicium présent dans la solution produite lors de la décomposition du matériau comprenant le silicium, pour former la solution contenant l'acide hexafluorosilicique.

L'invention est basée sur une évaporation sélective des espèces en présence dans l'échantillon, par laquelle on élimine du silicium et on garde les éléments que l'on souhaite doser. Le silicium peut alors être ramené à une concentration compatible avec d'autres techniques de dosage que l'AAS. Il est ainsi possible d'utiliser des techniques de dosage plus rapides comme l'ICPMS. Il a en outre été vérifié que l'appauvrissement en silicium obtenu par évaporation du tétrafluorure de silicium à l'aide du procédé selon l'invention peut s'accomplir sans perdre de quantité notable des éléments que l'on cherche à doser. Le procédé de dosage selon l'invention, reste donc très précis, fiable et très rapide lorsqu'il est combiné avec l'ICPMS. Par ailleurs combiné avec une technique AAS par exemple, ce procédé a pour mérite d'améliorer les seuils de détection.

Des aspects préférés, mais non limitatifs du procédé selon l'invention sont les suivants :

- l'étape de décomposition comprend une attaque du matériau avec l'agent d'attaque en phase liquide, auquel cas l'agent d'attaque comprend avantageusement un mélange d'acide nitrique et d'acide fluorhydrique ; dans ce cas, la réaction de décomposition est la suivante :

$$Si + HNO_3 + 6 \; HF \rightarrow H_2SiF_6 + NO_2 + H_2O$$

- alternativement, l'étape de décomposition comprend une attaque du matériau avec l'agent d'attaque en phase gazeuse.
- l'étape de dosage est réalisée par spectrométrie de masse à plasma couplé de manière inductive.
- au moins un élément à doser est compris dans la liste comprenant le sodium, le calcium, le potassium, l'aluminium, le fer, le cuivre, le nickel, le zinc, le manganèse, le magnésium, le chrome, le cobalt, le titane, le molybdène, le tungstène, le bore et le phosphore.
- la température atteinte dans l'étape de chauffage est de préférence comprise entre 70 et 100 °C, en étant de préférence voisine de 80 °C.
- le procédé comprend en outre, entre l'étape de chauffage et l'étape de dosage, une étape de mise à sec d'un volume de la solution pour dosage et de remise en solution du résidu sec. Le choix de la solution pour la remise en solution dépend de l'application visée par l'opérateur, et est fonction en particulier des composés et complexes qu'il souhaite former à partir des éléments à doser pour que ces composés et complexes soient solubles et stables après remise en solution. Ainsi, pour doser du fer, du chrome ou du sodium, la remise en solution est réalisée avantageusement avec de l'acide nitrique, typiquement à une concentration voisine de 0,1 %. Pour doser du bore ou du phosphore ,on utilise avantageusement de l'acide fluorhydrique, typiquement à une concentration voisine de 1 %. D'autres solutions peuvent s'avérer plus judicieuses pour doser d'autres éléments.

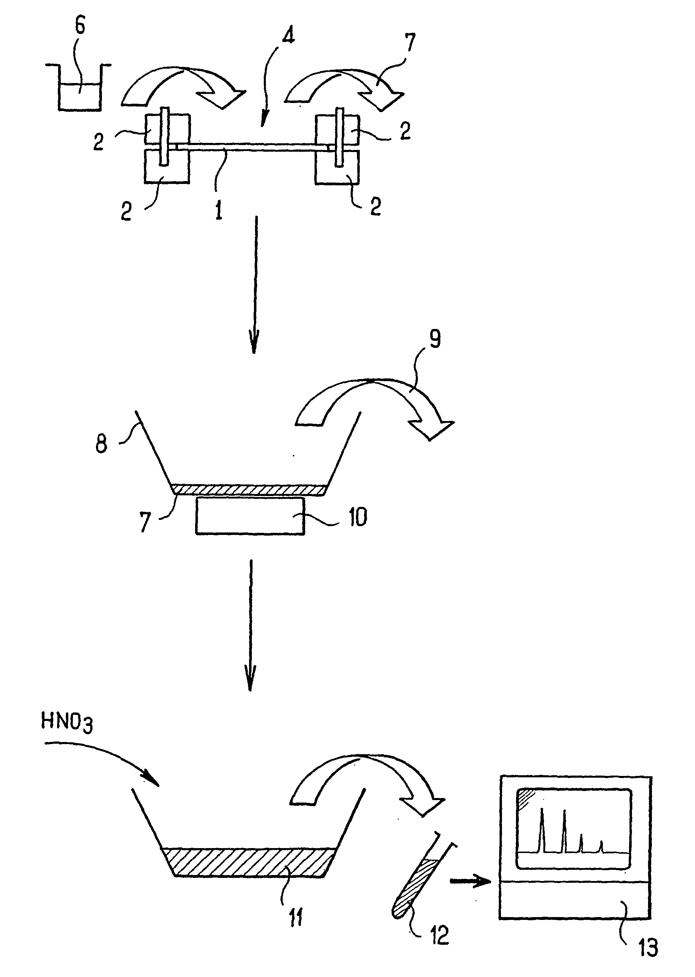

D'autres aspects, buts et avantages de l'invention seront mieux compris à l'aide de la description détaillée du mode de mise en oeuvre exposé ci-dessous. L'invention sera également mieux comprise à l'aide de la figure 1 unique. Cette figure représente schématiquement différentes opérations d'un exemple de mode de mise en oeuvre du procédé selon l'invention.

Cet exemple de mode de mise en oeuvre du procédé selon l'invention est décrit ci-dessous.

Ce mode de mise en oeuvre correspond à un dosage d'éléments contaminants dans un substrat 1 de silicium.

Comme représenté sur la figure 1, le substrat 1 est inséré entre deux pièces de Téflon® 2 pour former un réceptacle 4. Ce réceptacle permet de contenir une solution de gravure 6. Cette solution de gravure 6 correspond typiquement à un volume de 5 ml pour un substrat 1 de 125 mm de diamètre.

Cette solution de gravure 6 est un mélange d'acide nitrique ($HNO_3$) et d'acide fluorhydrique (HF). La concentration de l'acide nitrique dans cette solution de gravure 6 est de quelques dizaines de pour-cent. La concentration de l'acide fluorhydrique dans cette solution de gravure 6 est de quelques dizaines de pour-cent.

Après dissolution d'une certaine épaisseur du silicium du substrat 1, la solution ainsi obtenue par décomposition en phase liquide 7 est versée dans un récipient 8 de Téflon®, ou tout autre matériau équivalent choisi pour sa compatibilité avec les niveaux de contamination recherchés (polyéthylène, PTFE, etc.).

Avantageusement, la gravure est réalisée uniquement sur une épaisseur d'un micron environ à partir de la surface attaquée.

Le récipient 8 est ensuite porté, sur un élément chauffant 10, à une température inférieure à 100°C, pour permettre la formation de $SiF_4$ 9 et donc l'évaporation du silicium, selon la réaction

$$H_2SiF_6 \text{ (I)} \rightarrow SiF_4 \text{ (g)} + 2 \text{ HF}.$$

Cependant, il ne faut pas trop chauffer afin :

- de conserver une température inférieure à la température d'ébullition des éléments que l'on cherche à doser et de celles des composés ou complexes que ces éléments forment après décomposition et passage en solution ; et
- d'éviter l'éjection de micro gouttelettes, ce qui nuirait à la précision du dosage, notamment par la perte incontrôlée d'une certaine quantité d'éléments que l'on cherche à doser.

La température d'ébullition de l'acide fluorhydrique, dans les conditions normales de pression, étant de 112°C, l'évaporation de $SiF_4$ 9 doit donc être effectuée en dessous de cette température de 112°C.

Avantageusement, la température d'évaporation utilisée est comprise entre 70 et 100°C, et préférentiellement voisine de 80°C.

Typiquement les éléments que l'on cherche à doser sont le sodium, le calcium, le potassium, l'aluminium, le fer, le cuivre, le nickel, le zinc, le manganèse, le magnésium, le chrome, le cobalt, le titane, le molybdène, le tungstène, le bore et le phosphore.

Pour ces conditions, il a été vérifié que l'évaporation du silicium peut être réalisée sans pertes des contaminants que l'on cherche à doser.

La solution obtenue par décomposition en phase liquide 7 est ainsi évaporée jusqu'à la mise à sec, puis remise en solution en ajoutant 2 ml d'acide nitrique à 0,1 % au résidu sec pour obtenir une solution à analyser 11.

Avec un volume de 5 ml de solution obtenue par décomposition en phase liquide 7 et une remise en solution avec 1 ml d'acide nitrique, la concentration en silicium dans la solution à analyser 11 est inférieure au ppm. De plus, au cours de la mise en oeuvre décrite ci-dessus du procédé selon l'invention, on concentre les contaminants à doser d'un facteur 5, par rapport à la solution qui aurait été obtenue par des techniques de l'art antérieur, c'est à dire sans l'étape d'évaporation sélective selon l'invention.

Un volume de 1 ml de solution à analyser 11 est ensuite prélevée dans un tube 12 pour être soumis à une analyse par ICPMS 13. L'analyse par ICPMS 13 est rapide (temps typique de l'analyse 5 mn) et précise (limites de détection 100 ppt).

Il a été décrit ci-dessus un exemple de mode de mise en oeuvre du procédé selon l'invention grâce auquel on réalise un dosage d'éléments contaminants, dans un substrat de silicium. Mais on peut utiliser, de manière équivalente, le procédé selon l'invention, pour réaliser des dosages d'éléments dans des substrats de silicium sur isolant, des substrats de silicium oxydés en surface, du quartz, des éléments de quartzerie destinés aux fours de traitement thermique, etc..

On peut également, grâce au procédé selon l'invention doser des éléments contenus dans de l'oxyde de silicium, gravé par VPD.

Le procédé selon l'invention peut également être utilisé pour doser indirectement des éléments d'un autre matériau que le silicium. Par exemple, on peut disposer, pour doser les contaminants d'un four, une pièce d'un matériau comprenant du silicium, dans ce four. Les contaminants du four ou de cet autre matériau placé au voisinage migrent dans le silicium, et peuvent alors être dosés selon le procédé conforme à la présente invention.

Le procédé selon l'invention peut être utilisé non seulement pour doser des contaminants, par exemple métalliques, mais également pour doser des éléments dopants d'un matériau, tels que le bore ou le phosphore.

**Revendications**

1. Procédé de dosage d'au moins un élément dans un matériau (2) comprenant du silicium et utilisé dans la fabrication de substrats ou de composants pour l'optique, l'électronique ou l'optoélectronique, comprenant les étapes suivantes :

   - décomposer au moins une partie du matériau (2) avec un agent d'attaque (6) pour former une solution contenant de l'acide hexafluorosilicique (7) et les éléments à doser,
   - chauffer cette solution jusqu'à une température inférieure à la température d'ébullition de l'acide hexafluorosilicique, telle qu'au moins une partie substantielle de l'acide hexafluorosilicique (7) soit transformée en tétrafluorure de silicium (9) et qu'une partie substantielle du tétrafluorure de silicium s'évapore, pour obtenir une solution pour dosage dans laquelle la teneur en silicium soit sensiblement réduite tout en conservant la quantité de chaque élément à doser, et
   - doser au moins un élément contenu dans la solution pour dosage.

**2.** Procédé selon la revendication précédente, **caractérisé en ce que** la température atteinte dans l'étape de chauffage est comprise entre 70 et 100°C, en étant de préférence voisine de 80°C.

**3.** Procédé selon l'une des revendications précédentes, **caractérisé en ce que** l'étape de décomposition comprend une attaque du matériau avec l'agent d'attaque en phase liquide.

**4.** Procédé selon la revendication 3, **caractérisé en ce que** l'agent d'attaque comprend un mélange d'acide nitrique et d'acide fluorhydrique.

**5.** Procédé selon la revendication 1 ou 2, **caractérisé en ce que** l'étape de décomposition comprend une attaque du matériau avec l'agent d'attaque en phase gazeuse.

**6.** Procédé selon l'une des revendications précédentes, **caractérisé en ce que** l'étape de dosage est réalisée par spectrométrie de masse à plasma couplé de manière inductive.

**7.** Procédé selon l'une des revendications précédentes, **caractérisé en ce qu'**au moins un élément à doser est compris dans la liste comprenant le sodium, le calcium, le potassium, l'aluminium, le fer, le cuivre, le nickel, le zinc, le manganèse, le magnésium, le chrome, la cobalt, le titane, le molybdène, le tungstène, le bore et le phosphore.

**8.** Procédé selon l'une des revendications précédentes, **caractérisé en ce qu'**il comprend en outre, entre l'étape de chauffage et l'étape de dosage, une étape de mise à sec d'un volume de la solution pour dosage et de remise en solution du résidu sec.

**9.** procédé selon la revendication 8, **caractérisé en ce que** la remise en solution est réalisée avec une solution d'acide nitrique.

**10.** Procédé selon l'une des revendications 8 et 9, **caractérisé en ce que** la remise en solution est réalisée avec une solution d'acide fluorhydrique.

**Claims**

**1.** A method of assaying at least one element in a material (2) comprising silicon and used in the manufacture of substrates or components for optics, electronics, or optoelectronics, the method comprising the following steps:

- decomposing at least a portion of the material (2) with an etching agent (6) to form a solution containing hexafluorosilicic acid (7) and the elements to be assayed;
- heating said solution to a temperature lower than the boiling point for hexafluorosilicic acid, such that at least a substantial portion of the hexafluorosilicic acid (7) is transformed into silicon tetrafluoride (9) and a substantial portion of the silicon tetrafluoride evaporates, so as to obtain a solution for assaying in which the silicon content is significantly reduced while conserving the quantities of the elements to be assayed; and
- assaying at least one element contained in the solution for assaying.

**2.** A method according to the preceding claim, **characterized in that** the temperature reached in the heating step lies in the range 70°C to 100°C, and is preferably close to 80°C.

**3.** A method according to either preceding claim, **characterized in that** the decomposition step comprises etching the material with the etching agent in the liquid phase.

**4.** A method according to claim 3, **characterized in that** etching agent comprises a mixture of nitric acid and hydrofluoric acid.

**5.** A method according to claim 1 or 2, **characterized in that** the decomposition step comprises etching the material with the etching agent in the gas phase.

**6.** A method according to any preceding claim, **characterized in that** the assaying step is implemented by inductively coupled plasma mass spectrometry.

7. A method according to any preceding claim, **characterized in that** at least one element to be assayed is included in the list comprising: sodium; calcium; potassium; aluminum; iron; copper; nickel; zinc; manganese; magnesium; chromium; cobalt; titanium; molybdenum; tungsten; boron; and phosphorous.

8. A method according to any preceding claim, **characterized in that** it further comprises between the heating step and the assaying step a step of drying a volume of the solution for assaying and a step of putting the dry residue back into solution.

9. A method according to claim 8, **characterized in that** the residue is put back into solution with a solution of nitric acid.

10. A method according to claim 8 or claim 9, **characterized in that** the dry residue is put back into solution with a solution of hydrofluoric acid.


**Patentansprüche**

1. Verfahren zur Analyse mindestens eines Elements in einem Material (2), das Silizium enthält und bei der Herstellung von Substraten oder Komponenten für Optik, Elektronik oder Optoelektronik verwendet wird, das folgende Schritte umfaßt:

    - Zersetzen mindestens eines Teils des Materials (2) mit einem Angriffsmittel (6), um eine Lösung zu bilden, die Hexafluorokieselsäure (7) und die zu analysierenden Elemente enthält,
    - Erhitzen dieser Lösung bis auf eine Temperatur unter der Siedetemperatur der Hexafluorokieselsäure, so daß mindestens ein wesentlicher Teil der Hexafluorokieselsäure (7) in Siliziumtetrafluorid (9) umgesetzt wird und ein wesentlicher Teil des Siliziumtetrafluorids verdampft, um eine Analyselösung zu erhalten, in welcher der Siliziumgehalt deutlich verringert ist, wobei die Menge jedes zu analysierenden Elements erhalten bleibt, und
    - Analysieren mindestens eines in der Analyselösung enthaltenen Elements.

2. Verfahren nach dem vorstehenden Anspruch, **dadurch gekennzeichnet, daß** die in dem Erhitzungsschritt erzielte Temperatur zwischen 70 und 100°C und bevorzugt etwa um 80°C liegt.

3. Verfahren nach einem der vorstehenden Ansprüche, **dadurch gekennzeichnet, daß** der Zersetzungsschritt einen Angriff des Materials mit dem Angriffsmittel in flüssiger Phase umfaßt.

4. Verfahren nach Anspruch 3, **dadurch gekennzeichnet, daß** das Angriffsmittel eine Mischung aus Salpetersäure und Fluorwasserstoffsäure enthält.

5. Verfahren nach Anspruch 1 oder 2, **dadurch gekennzeichnet, daß** der Zersetzungsschritt einen Angriff des Materials mit dem Angriffsmittel in Gasphase umfaßt.

6. Verfahren nach einem der vorstehenden Ansprüche, **dadurch gekennzeichnet, daß** der Analyseschritt mittels Massenspektrometrie mit induktiv gekoppeltem Plasma durchgeführt wird.

7. Verfahren nach einem der vorstehenden Ansprüche, **dadurch gekennzeichnet, daß** mindestens ein zu analysierendes Element ausgewählt ist aus der Gruppe bestehend aus Natrium, Kalzium, Kalium, Aluminium, Eisen, Kupfer, Nickel, Zink, Mangan, Magnesium, Chrom, Kobalt, Titan, Molybdän, Wolfram, Bor und Phosphor.

8. Verfahren nach einem der vorstehenden Ansprüche, **dadurch gekennzeichnet, daß** es zwischen dem Erhitzungsschritt und dem Analyseschritt außerdem einen Schritt der Eintrocknung eines Volumens der Analyselösung und ein erneutes in Lösung bringen des trockenen Rückstands umfaßt.

9. Verfahren nach Anspruch 8, **dadurch gekennzeichnet, daß** das erneute in Lösung bringen mit einer Salpetersäure-Lösung vorgenommen wird.

10. Verfahren nach einem der Ansprüche 8 und 9, **dadurch gekennzeichnet, daß** das erneute in Lösung bringen mit einer Fluorwasserstoffsäure-Lösung vorgenommen wird.

HNO₃